# EUROPEAN PATENT SPECIFICATION

(11) **EP 3 801 231 B1**
(45) Date of publication and mention of the grant of the patent: **15.03.2023**
(21) Application number: 19743017.6
(22) Date of filing: 28.05.2019
(51) Int. Cl.: A61B 5/024, A61B 5/00

(54) **MEASURING DEVICE FOR MEASURING PHYSIOLOGICAL DATA OF A MAMMAL**
MESSVORRICHTUNG ZUR MESSUNG PHYSIOLOGISCHER DATEN EINES SÄUGETIERS
DISPOSITIF DE MESURE POUR LA MESURE DE DONNÉES PHYSIOLOGIQUES D'UN MAMMIFÈRE

(30) Priority: 28.05.2018 NL 2020993
(43) Date of publication of application: 14.04.2021
(73) Proprietor: Nedap N.V., 7141 DC Groenlo (NL)
(72) Inventor: MULLER, Roxie Sabri Romero, 7141 DC Groenlo (NL)
(74) Representative: V.O.
(86) International application number: PCT/NL2019/050306
(87) International publication number: WO 2019/231318

(56) References cited:
- WO-A1-2018/007272
- WO-A1-2018/022987
- US-A- 5 800 349
- US-A1- 2017 100 045

## Description

The invention relates to a measuring device for measuring physiological data of an animal such as a mammal for determining at least one condition parameter of the animal, comprising a first and second module (12, 14) which are configured to be worn, in use, by the animal, wherein the first and second module are furthermore configured for them, from a condition of being mechanically separated from each other, to be mechanically connected with each other whereby in the connected condition, in use, between the first and second module is a skin part of the animal, wherein one of the modules comprises a source (122) for emitting electromagnetic radiation such as light (L) and wherein another of the modules comprises a sensor system (142) for measuring a parameter such as intensity of a fraction (Lm) of the emitted radiation (L) received via that skin part and for generating a measuring signal (S_{L}) which is indicative of the measured parameter.

A device of this type is known from NL2015582 or US 2017/100045 A1.

A problem with such a device is that, in use, the source of one of the modules is not always optimally positioned with respect to the sensor system of the other module. According to the prior art, the first and second module are attached to each other by means of a pin/hole connection whereby, after the first and second modules have been connected with each other, the first and second module can be rotated relative to each other around an axial axis of the pin. Even if the first and second module are attached to each other such that the source is situated approximately opposite the sensor system, it can still happen that, in use, the first and second module rotate relative to each other, for instance in that someone inadvertently touches either of the parts, or in that the first and second module in the process of being attached to each other are still rotated relative to each other unnoticed.

Object of the invention is to provide a solution to the problem mentioned. To that end, the measuring device according to the invention is characterized in that the measuring device is configured such that, in use, the first and second module can possibly have been connected or be connected (stably) with each other only when they have at least one mutual orientation that falls within a predetermined range of mutual orientations according to independent claim 1.

Due to there being at least one mutual orientation where the first and second module can be connected with each other, it is only necessary, during connecting, to choose the right orientation where the first and second module can be connected with each other whereby in connected condition the sensor system and the source are situated opposite each other. Once the first and second module have been connected with each other in the correct manner, the position of the sensor system with respect to the source is maintained, remaining at least substantially unchanged.

In particular, it holds that the first and second module can possibly have been connected or be connected (stably) with each other only when they have one mutual orientation that falls within a predetermined range of mutual orientations according to claim 1.

In this manner, also when mechanically interconnecting the first module with the second module, no mistakes can be made anymore and it will always be ensured that the source is situated opposite the sensor system. The above-mentioned range of mutual orientations may be a consequence of tolerances but may also have been provided intentionally so that connecting the first module with the second module does not require absolute precision. It is only necessary that the first module and second module within a limited range take up the proper orientation with respect to each other for them to be connected with each other.

It holds here that the range is limited in a possible limited turning around an axis which is at least substantially perpendicular to a plane which, in use, extends parallel to sides of the first and second module that face each other.

More particularly, it holds here that the limited turning involves a maximum angle A, where A is less than 15 degrees, preferably less than 10 degrees and more preferably less than 5 degrees.

In particular, it holds furthermore that the measuring device is configured such that if the first and second module, in use, have been attached to each other, the first and second module can move relative to each other in a possible limited translation along an axis which is at least substantially perpendicular to a plane which, in use, extends parallel to sides of the first and second module that face each other. It is thus ensured that also with varying thicknesses of the skin part, the first and second module can be comfortably attached to the animal.

According to a practical embodiment, it holds that the second module is provided with a lead-through opening and the first module is provided with a projecting pin, while, in use, the pin extends through the opening.

In addition to this, it holds in particular that an inner surface of the opening and an outer surface of the pin have a shape such that when an axial axis of the pin and an axial axis of the opening coincide, the pin can have been or be inserted into the opening only within at least one limited continuous range of rotation angles around its axial axis and with respect to the opening.

According to a practical embodiment thereof, it holds that the outer surface is provided with at least one groove which extends in a length direction of the pin while the inner surface is provided with at least one ridge which, in use, extends in the groove and/or that the inner surface is provided with at least one groove which extends in a length direction of the opening while the outer surface is provided with at least one ridge which extends in the length direction of the pin and which, in use, extends in the groove.

For the foregoing exemplary embodiments, it holds that when the first and second module have been connected with each other according to the one possibility or one of the possibilities, the sensor system and the source are situated at least substantially opposite each other, such that radiation from the source is directed to the sensor system (142). As mentioned, it holds preferably that the first and second module can be connected with each other only according to one possibility, so that, also during the actual connecting, that is, during the fitting to the animal, no mistakes can be made and the sensor system and source are always situated at least substantially opposite each other when the first and second module have been connected with each other. Approximately right positioning with respect to each other can be a consequence of tolerances to which the first and second module can be manufactured, but can also be a deliberate result of the design of the measuring device. In the latter case, it is thereby accomplished that, in use, upon attachment to the animal, it is only necessary that the first and second module, when being fitted to the animal, are positioned with respect to each other approximately in the right manner, which does not require absolute infinite precision.

In particular, the measuring device is intended, with a relatively long useful life, to make measuring physiological data, such as the heartbeat of the mammal, possible.

In realization of this, the measuring device provides in particular the possibility of measuring physiological data of a mammal for determining at least one condition parameter of the mammal. The measuring device accordingly comprises a measuring device to be worn by the mammal, having a first module and second module to be disposed mutually on opposite sides opposite a skin part of the mammal. The first module comprises the source for generating the radiation. The second module comprises the sensor unit for measuring an intensity of a fraction of the radiation received via that skin part and delivering a measuring signal which is indicative of the measured value of the radiation. The measuring device is preferably configured for pulsewise activating the source, while the measuring signal is indicative of the value of the intensity measured during the pulsewise activation. The measuring device may furthermore be provided with synchronization means for synchronously activating the source and the second module, while the synchronization means comprise an energy transmitting unit which is part of one of the first and the second module, and comprise a detector which is part of the other one of the first and the second module, while in an operating condition the energy transmitting unit pulsewise generates an electromagnetic field, and the detector receives this field and generates therefrom a supply voltage for use in that other module.

The at least one condition parameter to be determined is, for example, a heartbeat of the animal or a blood pressure of the animal. Also, the condition parameter to be determined can be a blood value, i.e., an indication of a concentration of one or more constituents in the blood. Also, a combination of condition parameters can be established with the measuring device and/or the measuring method.

As in the measuring device the radiation used for the measurement is preferably generated pulsewise, the intensity of the generated radiation can be raised without thereby also increasing the electrical energy consumption. Consequently, raising the intensity used does not need to be accompanied by a decrease of the useful life.

The invention will now be further explained on the basis of the drawings, in which:
Fig. 1 shows a possible embodiment of a measuring device according to the invention;
Fig. 2 shows a view of the second module in the direction of the arrow P₁ of Fig. 1;
Fig. 3 shows a view of the second module in the direction of the arrow P₂ of Fig. 1;
Fig. 4 shows a system according to the invention comprising a measuring device according to the invention and a tool;
Fig. 5 shows the system of Fig. 4 when the first and second module have been attached to the tool;
Fig. 6 shows the manner in which the first module can be attached to the tool;
Fig. 7 schematically shows the measuring device of Fig. 1;
Fig. 8 schematically shows components of the measuring device of Fig. 1;
Fig. 9 schematically shows an alternative embodiment of an opening and pin of the device of Fig. 1;
Fig. 10 schematically shows an alternative embodiment of an opening and pin of the device of Fig. 1.

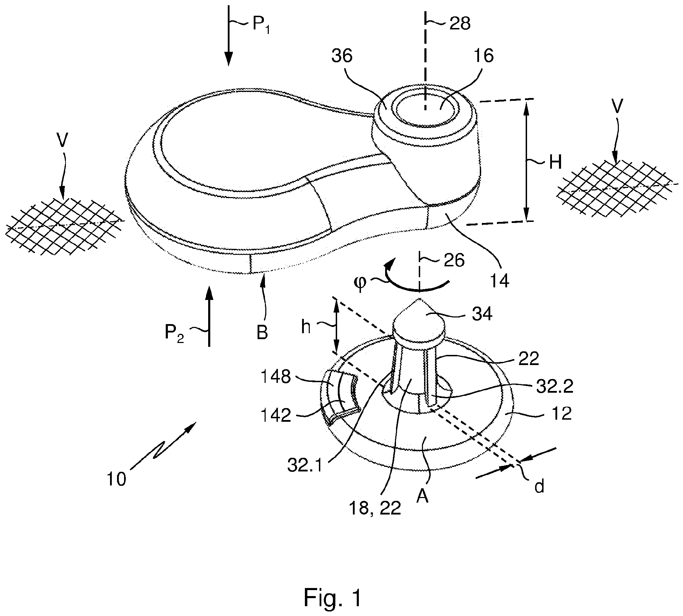

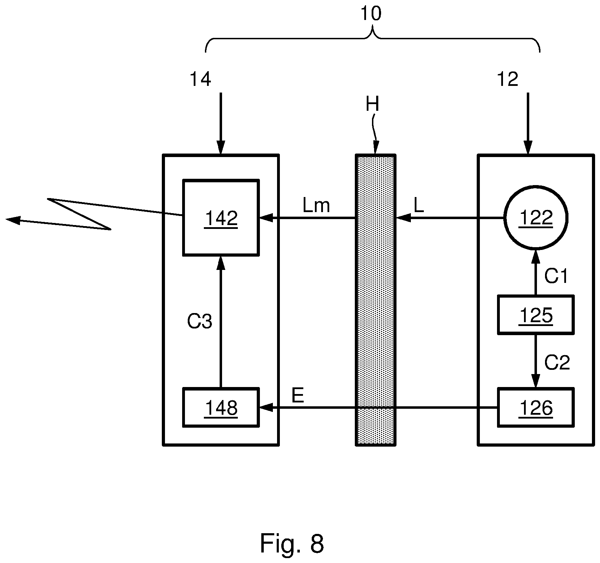

In Figure 1, with reference numeral 10 an embodiment of a measuring device according to the invention is designated. The measuring device is configured for measuring physiological data of an animal, such as a mammal, for determining at least one condition parameter of the animal. The device comprises a first module 12 and a second module 14 which are configured to be worn, in use, by the animal. The first and second module are configured for them, from a condition of being mechanically separated from each other as shown in Figure 1, to be connected with each other, whereby in connected condition, in use, between the first and second module is a skin part of the animal, as is also shown in Figure 8.

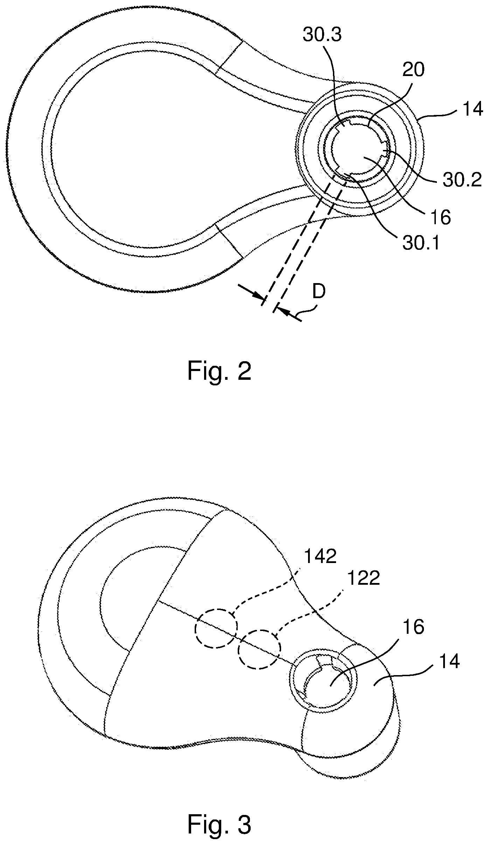

One of the two modules comprises a source 122 for emitting electromagnetic rays such as light. In this example, this involves the second module (see Fig. 3). Another one of the modules, in this example the first module 12, is provided with a sensor system 142 for measuring a parameter such as an intensity of a fraction (Lm) of the emitted radiation (L) received via the skin part, and for generating a measuring signal (S_{L}) which is indicative of the measured parameter.

The measuring device 10 is configured such that, in use, the first and second module can possibly have been connected (stably) with each other only when they have at least one predetermined mutual orientation or when they have at least one mutual orientation that falls within a predetermined range of mutual orientations.

In Figure 1, there is shown the mutual orientation of the first and second module where they can be connected with each other when the first and second module are moved towards each other in the direction of the arrow P₁.

In this example, it holds that the second module is provided with a lead-through opening 16 and the first module 12 is provided with a projecting pin 18, this pin, in use, extending through the opening 16. This situation is shown in Figure 8.

An inner surface 20 of the opening 16 and an outer surface 22 of the pin 18 have a shape such that when an axial axis 26 of the pin 18 and an axial axis 28 of the opening 16 coincide, the pin can have been or be inserted into the opening only within at least one (in this example three) limited continuous range of rotation angles φ around its axial axis and with respect to the opening.

In this example, this has been achieved in that the inner surface 20 is provided with three grooves 30.1 - 30.3. Further, the outer surface of the pin 18 is provided with three ridges 32.1 - 32.3 which extend in the length direction of the pin and which, in use, respectively extend in the grooves 30.1 - 30.3. In this example, this means that the pin can be slid into the opening 16 in three rotational positions, indicated with the angle φ. These mutual positions differ by 120 degrees. Preferably, however, it holds that the ridges 32.2 and 32.3 as well as the grooves 30.2 and 30.3 are omitted, so that the pin can only be slid into the opening in one rotational position of the angle φ.

If a ridge has a thickness d as shown in Figure 1 somewhat smaller than a width D of a groove as shown in Figure 2, this has as a consequence that when the pin has been slid into the opening, it can rotate relative to the opening in a limited continuous range of rotation angles around an axial axis. This has as an advantage that when the pin is being fitted into the opening the ridges and grooves do not need to be perfectly aligned with each other for the pin to be slid into the opening. This applies both to the variant where the opening is provided with three grooves and the pin is provided with three ridges, the ridges being received in the respective grooves, and to the variant where the opening is only provided with a single groove 30.1 and the pin is provided with a corresponding single ridge 32.1. It holds, then, that the first and second module can possibly have been connected with each other only when they have a predetermined mutual orientation. In that case, the width of a groove is approximately equal to the width of a ridge. Or when they have a mutual orientation that falls within a predetermined range of mutual orientations (in that case, the width D of a groove is a bit greater than the thickness d of a ridge). Preferably, it holds in that case that the range is limited in a possible limited turning around the axis which is at least substantially perpendicular to a plane V which, in use, extends parallel to mutually facing sides A and B of the first and second module, respectively. This axis has the same direction as an axial axis 26 of the pin and an axial axis 28 of the opening. The above-mentioned limited turning is due to the ridge having a thickness less than the width of the groove, and involves for example a maximum angle in the direction of the angle φ, being less than 15 degrees, preferably less than 10 degrees and more preferably less than 5 degrees.

It holds furthermore that the measuring device is configured such that if the first and second module, in use, have been attached to each other, the first and second module can move relative to each other in a possible limited translation along an axis which is at least substantially perpendicular to a plane V which, in use, extends parallel to sides of the first and second module that face each other.

This limited movement is determined by the difference between a height H of the opening 16 and the height h of the pin up to a knob 34 of the pin. The knob 34 of the pin has a diameter a bit greater than that of the opening 16, such that the knob 34, due to its being made of slightly flexible material, can be forced through the opening 16, whereupon the knob is above an outer surface 36 of the second module. The difference between the height H and the height h determines the distance over which, in use, the first and second module can move relative to each other in the direction of the axial axes 26 and 28. In other words, they can move relative to each other in a possible limited translation along an axis which is, at least substantially, perpendicular to the plane V mentioned.

The possible limited translation is B centimeters at a maximum, where B is less than 6 cm, preferably less than 4 cm, and more preferably less than 2 cm.

In the example shown in Figures 1 to 3, then, the first and second module can be connected with each other in three different rotational positions with respect to each other. For one of these positions, then, it holds that the sensor system 142 and the source 122 are situated, at least substantially, opposite each other, such that the radiation from the source is directed to the sensor system. If the grooves 30.2 and 30.3 as well as the ridges 32.2 and 32.3 are omitted, it holds that the first and second module can be connected with each other in one manner only, whereby in that case, in connected condition, the sensor system and the source are situated, at least substantially, opposite each other, such that the radiation of the source is directed at the sensor system.

Preferably, it holds that the measuring device is designed for pulsewise activating the light source, wherein the measuring signal is indicative of the value of the intensity measured during the pulsewise activation, wherein the measuring device is furthermore provided with synchronization means for synchronously activating the light source and the second module (14), wherein the synchronization means comprise an energy transmitting unit (126) which is part of one of the first and the second module and comprise a detector (148) which is part of the other one of the first and the second module, wherein in an operating condition the energy transmitting unit (126) pulsewise generates an electromagnetic field, and the detector receives this field and generates therefrom a supply voltage for use in that other module.

All this will be explained in more detail hereinafter. At this point, however, it is relevant to mention that when the first and second module have been connected with each other according to the one possibility or one of the possibilities, the energy transmitting unit and the detector are situated at least substantially opposite each other, such that the radiation of the energy transmitting unit is directed to the detector.

Again, it holds that when there are three possibilities of attaching the first and second module to each other, it holds only for the possibility where the ridge 32.1 is received in the groove 30.1, the ridge 32.2 is received in the groove 30.2, and the ridge 32.3 is received in the groove 30.3, that the energy transmitting unit and the detector are situated, at least substantially, opposite each other. Again, it holds that if the ridges 32.2 and 32.3 as well as the grooves 30.2 and 30.3 are omitted, there is only one possibility of connecting the first and second module with each other, whereby in this possibility the energy transmitting unit and the detector are situated opposite each other.

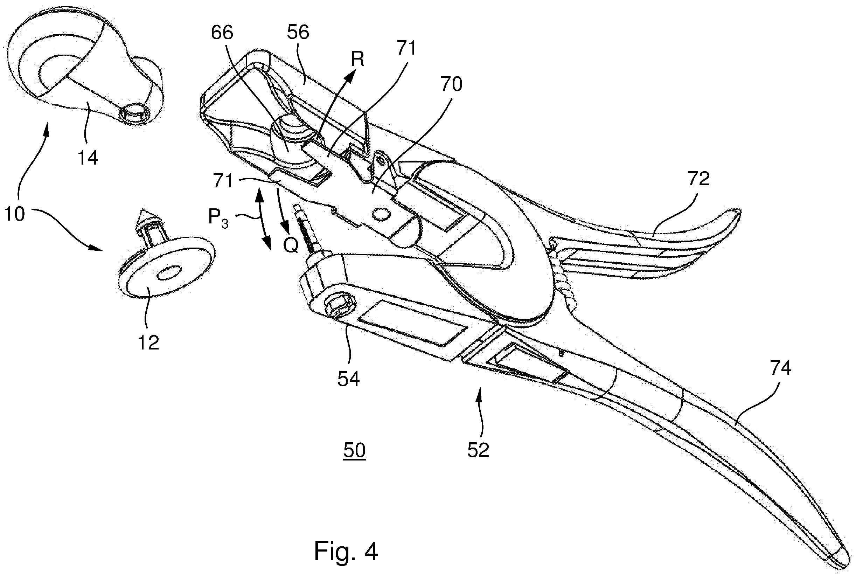

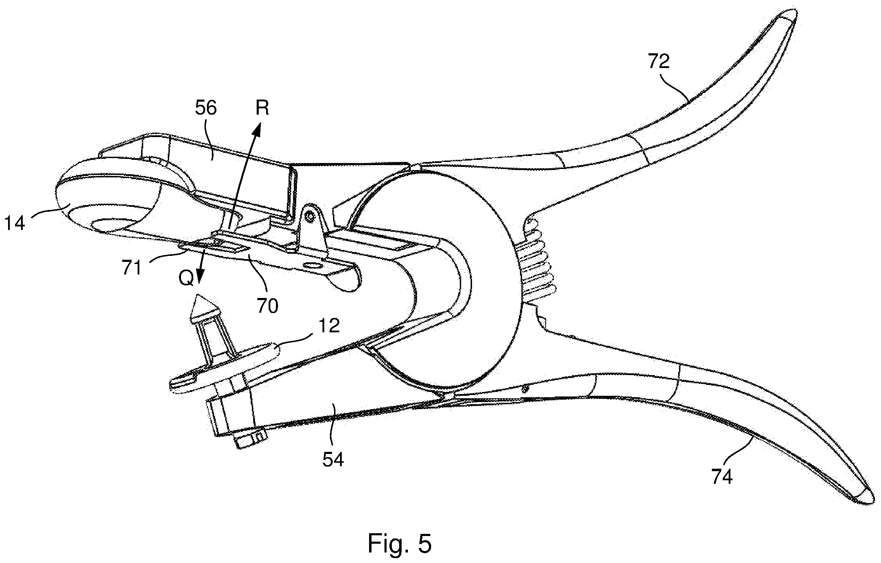

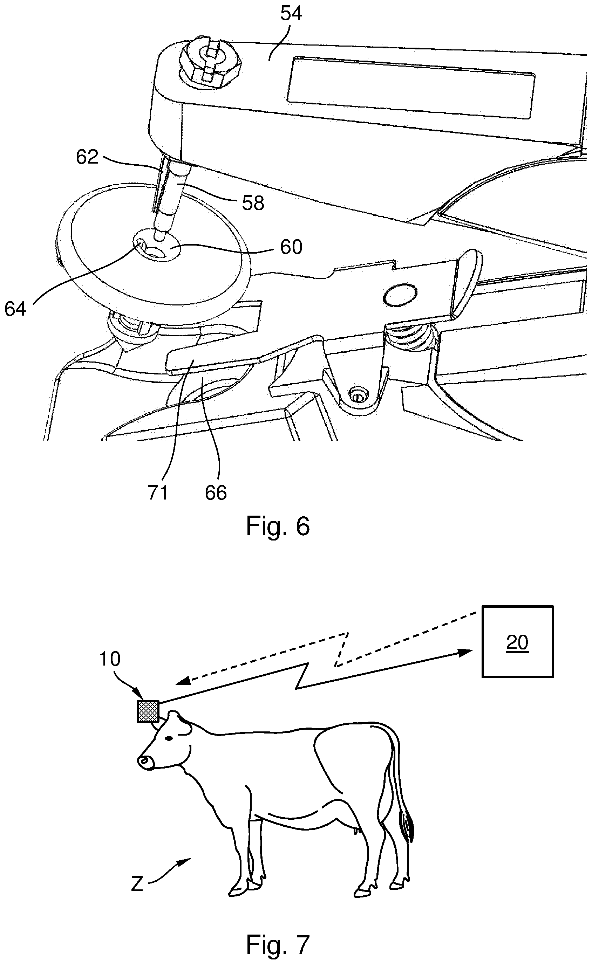

Presently, with reference to Figures 4-6, a possible embodiment of a system according to the invention will be discussed. The system comprises a measuring device 10 including the first module 12 and the second module 14. The system further comprises a tool 52 including a first part 54 and a second part 56 which are connected movably relative to each other. Viewed from the first part 54, the second part 56 can move along a predetermined path P₃ towards the first part and away from the first part. The system is configured such that the first module 12 can be mechanically connected with the first part in an unequivocal manner. To this end, the tool is provided with a securing pin 58 (see Fig. 6) and the first module 12 is provided with a securing opening 60 in which the securing pin can be inserted for securing the first module to the tool. The securing opening and the securing pin have a complementary shape so that the first module and the tool can be mechanically connected with each other in one manner only.

In this example, this has been realized by providing the securing pin 58 with a ridge 62 and providing the securing opening with a groove 64.

In this example, it holds that the securing opening 60 extends into the pin 18 of a first module to enable the first module to be mechanically connected with the tool in the unequivocal manner. The tool is furthermore provided with a hollow space 66 partly open to the outside, in which a second module can be received for mechanically connecting the second module with the tool in an unequivocal manner. The hollow space 66 and a part of an external side of the second module 14 have a complementary shape so that the second module and the tool can be mechanically connected with each other in one manner only. In this example, the second module 14 can be manipulated in the hollow space 66 and be retained, by means of a spring biased clip 70 and an end 71 of which can be moved by hand in the direction Q so that the second module can be received in the space 66 and of which the free end upon release is moved in the direction R so that the second module is retained in the hollow space 66.

The tool in this example is implemented as a pair of pliers where the first and second part can be moved towards each other by moving two handles 72, 74 respectively towards each other. When the handles 72 and 74 are moved towards each other, the pin 18 will automatically end up in the opening 16, that is, such that the ridge 32.1 is slid into the groove 30.1, the ridge 32.2 is slid into the groove 30.2, and the ridge 32.3 is slid into the groove 30.3. It is noted here that presently it does not matter anymore that in the embodiment in which the first module has three ridges and the second module has three grooves the first and second module can be attached to each other in three different ways (in each case with a difference in rotational position of 120 degrees around the axial axis of the pin), because by the use of the tool only one way of attachment is chosen. As a result of the grooves taking up the ridges, a condition of the first and the second module being stably connected with each other is involved. Accordingly, 'stably' is here understood to mean that, after connection, still some limited rotation is possible because of tolerances, or in that this has been determined beforehand and that also in a direction perpendicular to the plane V, that is, in a direction of the axial axis of the pin 18, still a limited translation is possible of the first module relative to the second module to allow the measuring device to be comfortably fitted on the animal.

In Figure 7 the measuring device is shown in use. Presently, it will be set out how the electronics of the measuring device may be implemented. This is only an exemplary embodiment which stands apart from the idea regarding the manner in which the first and second module can be connected with each other.

As further shown schematically in Figure 8, the first module 12 includes a light source 122 for generating light L, an energy transmitting unit 126, and a control unit 125 for pulsewise activating the light source 122 and the energy transmitting unit 126.

The second module 14 is provided with a sensor unit 142 and a detector 148. The control unit 125, the energy transmitting unit 126 and the detector 148 jointly form synchronization means for pulsewise activating the sensor unit 142 synchronously with the light source 122. In the first module 12 a battery or other energy source (not shown) is included which supplies the electrical energy for the light source 122, the control unit 125 and the energy transmitting unit 126. The light source 122 is activated with control signal C1. The energy transmitting unit 126, upon activation by the control unit 125 with control signal C2, generates an electromagnetic field E. The detector 148 included in the second module 14 receives this electromagnetic field and generates therefrom a supply voltage, and also control signal C3 with which the sensor unit 142 is driven. In the embodiment shown, the sensor unit 142 and the light source 122 are synchronously activated with the aid of the energy transmitting unit 126 and the detector 148. In other words, the points of time at which the sensor unit 142 and the light source 122 are activated are mutually correlated. As a result, measuring can be done reliably also with a relatively short pulse duration. Moreover, the light source 122 and the sensor unit 142 can be fed from a common energy source. The activation points of time can coincide, but alternatively one of the activation points of time may be shifted by a predetermined time interval relative to the other activation point of time. It may be, for instance, that the sensor unit 142 has a start-up time between the moment of receiving an activation signal C3 from the detector 148 and the moment at which it becomes actually operational. It may also be that the sensor unit 142 after detecting the light pulse Lm needs some time for processing, storage or forwarding information. This can be allowed for in an embodiment in which the control unit 125 activates the control signal C2 in a time interval that begins before the time interval of activating the control signal C1 and that ends following the time interval of activating the control signal C1. Another solution would be for the light source 122 to be activated during a time interval that is long enough for the sensor unit to function properly. This provides the advantage that control is simpler. However, the energy consumption by the light source 122 is higher then.

In the embodiment shown, the light source 122 is an infrared LED or OLED, but if desired an LED may be used for generating light in a different part of the light spectrum, for example, in the visible range, or in the ultraviolet range. Also, conceivably, a different type of light source is used. An LED or OLED, however, is the most suitable for this purpose since this type of light source can be easily driven at relatively low voltages and with a short pulse duration.

The second module 14 has a sensor unit 142 for measuring an intensity of a fraction Lm of the generated light L, received via that skin part, for example the auricle.

The measured intensity depends on *inter alia* the extent to which light en route from the light source 122 to the sensor unit 142 is absorbed by the blood in the veins and capillaries in that skin part H. This, in turn, depends on *inter alia* the diameter of the veins and capillaries. The diameter varies periodically with the frequency of the heartbeat. Hence, also the measured intensity varies with that same frequency. In addition, depending on the wavelength of the light, the absorption may depend to a greater or lesser extent on the concentration of constituents in the blood. The sensor unit can thus determine the frequency of the heartbeat as a condition parameter of the mammal from the measured intensity variations.

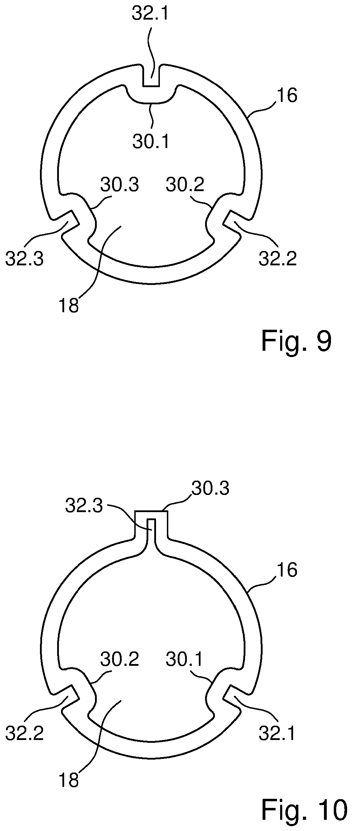

The invention is not limited to the embodiments described. Thus, the ridges 32.1-32.3 may be provided at an inner side of the opening 16 while the grooves 30.1-30.3 are provided on an outer side of the pin. In use, again, the ridges are received in the grooves as discussed above (Fig. 9). Also, an inner side of the opening may be provided with ridges and grooves while an outer side of the pin is provided with grooves and ridges. In use, the ridges of the pin then fall into the grooves of the opening and the ridges of the opening then fall into the grooves of the pin (Fig. 10). Also, it is possible for the first and second module to be provided with self-locating means which provide that the first and second module when being connected are turned relative to each other in a direction that corresponds with the at least one predetermined mutual orientation or with the predetermined range of mutual orientations. Thus, in a direction in which the pin 18 is inserted into the opening, the grooves may for instance taper to obtain the self-locating effect and hence the self-locating means. As an alternative, it is possible for the ridges to taper in a direction in which the pin 18 is inserted into the opening to obtain the self-locating effect and hence the self-locating means. The invention is limited by the scope of the appended claims.

## Claims

1. A measuring device for measuring physiological data of an animal such as a mammal for determining at least one condition parameter of the animal, comprising a first and second module (12, 14) which are configured to be worn, in use, by the animal, wherein the first and second module are furthermore arranged to be mechanically connected with each other, from a condition of being mechanically separated from each other, whereby in the connected condition, in use, between the first and second module is a skin part of the animal, wherein one of the modules comprises a source (122) for emitting electromagnetic radiation such as light (L) and wherein another of the modules comprises a sensor system (142) for measuring a parameter such as intensity of a fraction (Lm) of the emitted radiation (L) received via that skin part and for generating a measuring signal (S_{L}) which is indicative of the measured parameter, wherein the measuring device is configured such that, in use, the first and second module can be connected stably with each other only when they have at least one mutual orientation that falls within a predetermined range of mutual orientations, **characterized in that** the range is limited in a limited turning around an axis which is at least substantially perpendicular to a plane which, in use, extends parallel to sides of the first and second module that face each other.

2. The measuring device according to claim 1, **characterized in that** the first and second module can possibly have been connected or be connected (stably) with each other only when they have one mutual orientation that falls within the predetermined range of mutual orientations.

3. The measuring device according to claim 1 or 2, **characterized in that** the limited turning involves a maximum angle A where A is less than 15 degrees, preferably less than 10 degrees and more preferably less than 5 degrees.

4. The measuring device according to any one of the preceding claims, **characterized in that** the measuring device is configured such that if the first and second module, in use, have been attached to each other, the first and second module can move relative to each other in a possible limited translation along an axis which is at least substantially perpendicular to a plane which, in use, extends parallel to sides of the first and second module that face each other.

5. The measuring device according to claim 4, **characterized in that** the possible limited translation is B cm at a maximum, where B is less than 6 cm, preferably less than 4 cm, and more preferably less than 2 cm.

6. The measuring device according to any one of the preceding claims, **characterized in that** the first and second module are provided with self-locating means which provide that in connecting the first and second module the first and second module are turned relative to each other in a direction which corresponds to the predetermined range of mutual orientations.

7. The measuring device according to any one of the preceding claims, **characterized in that** the second module is provided with a lead-through opening and the first module is provided with a projecting pin, while, in use, the pin extends through the opening.

8. The measuring device according to claim 7, **characterized in that** an inner surface of the opening and an outer surface of the pin have a shape such that when an axial axis of the pin and an axial axis of the opening coincide, the pin can have been or be inserted into the opening only within at least one limited continuous range of rotation angles around its axial axis and with respect to the opening.

9. The measuring device according to claim 8, **characterized in that** the range is A, where A is less than 15 degrees, preferably less than 10 degrees, and more preferably less than 5 degrees.

10. The measuring device according to claim 8 or 9, **characterized in that** the outer surface is provided with at least one groove which extends in a length direction of the pin while the inner surface is provided with at least one ridge which, in use, extends in the groove and/or that the inner surface is provided with at least one groove which extends in a length direction of the opening while the outer surface is provided with at least one ridge which extends in the length direction of the pin and which, in use, extends in the groove.

11. The measuring device according to any one of the preceding claims, **characterized in that** when the first and second module have been connected with each other according to the one possibility or one of the possibilities, the sensor system (142) and the source (122) are situated at least substantially opposite each other, such that radiation from the source is directed to the sensor system (142).

12. The measuring device according to any one of the preceding claims, **characterized in that** the measuring device is configured for pulsewise activating the light source, wherein the measuring signal is indicative of the value of the intensity measured during the pulsewise activation, wherein the measuring device is furthermore provided with synchronization means for synchronously activating the light source and the second module (14), wherein the synchronization means comprise an energy transmitting unit (126) which is part of one of the first and the second module and comprise a detector (148) which is part of the other one of the first and the second module, wherein in an operating condition the energy transmitting unit (126) pulsewise generates an electromagnetic field, and the detector receives this field and generates therefrom a supply voltage for use **in that** other module.

13. The measuring device according to claim 12, **characterized in that** the measuring device is configured such that, in use, the synchronization means (125) pulsewise activates the light source (122) and the energy transmitting unit (126), wherein the detector (148) pulsewise activates the sensor means (142) in response to the energy received from the energy transmitting unit (126) or that the synchronization means (145) pulsewise activates the sensor means (142) and the energy transmitting unit (126), wherein the detector (148) pulsewise activates the light source (122) in response to the energy received from the energy transmitting unit (126).

14. The measuring device according to claim 12 or 13, **characterized in that** when the first and second module have been connected with each other according to the one possibility or one of the possibilities, the energy transmitting unit and the detector are situated at least substantially opposite each other, such that radiation from the energy transmitting unit is directed to the detector.

15. A system comprising a measuring device according to any one of the preceding claims, **characterized in that** the system comprises a tool including a first part and a second part which are connected movably relative to each other, wherein, viewed from the first part, the second part can move along a predetermined path towards the first part and away from the first part, the system being configured such that the first module can be mechanically connected to the first part in one unequivocal manner and the second module can be mechanically connected to the second part in one unequivocal manner, wherein if the first part and the second part are moved towards each other the first and second module are (stably) connected with each other while they have the predetermined mutual orientation; or have the mutual orientation that falls within the predetermined range of mutual orientations.

16. The system according to claim 15, **characterized in that** the tool is implemented as a pair of pliers of which the first part and the second part can be moved towards each other by moving two handles of the pliers towards each other.

17. The system according to claim 15 or 16, **characterized in that** the tool is provided with a securing pin and the first module is provided with a securing opening in which the securing pin can be inserted for securing the first module to the tool.

18. The system according to claim 17, **characterized in that** the securing opening extends into the pin of the first module according to claim 5 for mechanically connecting the first module with the tool in the unequivocal manner.

19. The system according to claim 18, **characterized in that** the securing opening and the securing pin have a complementary shape so that the first module and the tool can be mechanically connected with each other in one manner only.

20. The system according to any one of claims 15-19, **characterized in that** the tool is provided with a hollow space partly open to the outside in which the second module can be received for mechanically connecting the second module with the tool in the unequivocal manner.

21. The system according to claim 20, **characterized in that** the hollow space and a part of an outer side of the second module have a complementary shape so that the second module and the tool can be mechanically connected with each other in one manner only.

## Patentansprüche

1. Messvorrichtung zum Messen physiologischer Daten eines Tieres, wie eines Säugetieres, zum Bestimmen wenigstens eines Zustandsparameters des Tieres, umfassend ein erstes und ein zweites Modul (12, 14), die so ausgelegt sind, dass sie im Gebrauch von dem Tier getragen werden, wobei das erste und das zweite Modul ferner so angeordnet sind, dass sie aus einem Zustand, in dem sie mechanisch voneinander getrennt sind, mechanisch miteinander verbunden werden, wobei sich im verbundenen Zustand im Gebrauch zwischen dem ersten und dem zweiten Modul ein Hautteil des Tieres befindet, wobei eines der Module eine Quelle (122) zum Emittieren von elektromagnetischer Strahlung wie Licht (L) umfasst und wobei ein anderes der Module ein Sensorsystem (142) zum Messen eines Parameters wie Intensität eines Anteils (Lm) der emittierten Strahlung (L), die über diesen Hautteil empfangen wird, und zum Erzeugen eines Messsignals (S_{L}), das den gemessenen Parameter angibt, umfasst, wobei die Messvorrichtung so ausgelegt ist, dass, im Gebrauch das erste und das zweite Modul nur dann stabil miteinander verbunden werden können, wenn sie wenigstens eine gegenseitige Ausrichtung haben, die in einen vorbestimmten Bereich gegenseitiger Ausrichtungen fällt, **dadurch gekennzeichnet, dass** der Bereich durch eine begrenzte Drehung um eine Achse begrenzt ist, die wenigstens im Wesentlichen senkrecht zu einer Ebene ist, die sich im Gebrauch parallel zu Seiten des ersten und des zweiten Moduls erstreckt, die einander gegenüberliegen.

2. Messvorrichtung nach Anspruch 1, **dadurch gekennzeichnet, dass** das erste und das zweite Modul möglicherweise nur dann (stabil) miteinander verbunden worden sind oder miteinander verbunden werden können, wenn sie eine gegenseitige Ausrichtung haben, die in den vorbestimmten Bereich der gegenseitigen Ausrichtungen fällt.

3. Messvorrichtung nach Anspruch 1 oder 2, **dadurch gekennzeichnet, dass** die begrenzte Drehung einen maximalen Winkel A umfasst, wobei A weniger als 15 Grad, vorzugsweise weniger als 10 Grad und bevorzugter weniger als 5 Grad beträgt.

4. Messvorrichtung nach einem der vorhergehenden Ansprüche, **dadurch gekennzeichnet, dass** die Messvorrichtung so ausgelegt ist, dass, wenn das erste und das zweite Modul im Gebrauch aneinander befestigt worden sind, das erste und das zweite Modul sich relativ zueinander in einer möglichen begrenzten Verschiebung entlang einer Achse bewegen können, die wenigstens im Wesentlichen senkrecht zu einer Ebene ist, die sich im Gebrauch parallel zu den Seiten des ersten und des zweiten Moduls erstreckt, die einander gegenüberliegen.

5. Messvorrichtung nach Anspruch 4, **dadurch gekennzeichnet, dass** die mögliche begrenzte Verschiebung maximal B cm beträgt, wobei B weniger als 6 cm, vorzugsweise weniger als 4 cm und bevorzugter weniger als 2 cm beträgt.

6. Messvorrichtung nach einem der vorhergehenden Ansprüche, **dadurch gekennzeichnet, dass** das erste und das zweite Modul mit Selbstlokalisierungsmitteln versehen sind, die sicherstellen, dass beim Verbinden des ersten und des zweiten Moduls das erste und das zweite Modul relativ zueinander in eine Richtung gedreht werden, die dem vorbestimmten Bereich der gegenseitigen Ausrichtungen entspricht.

7. Messvorrichtung nach einem der vorhergehenden Ansprüche, **dadurch gekennzeichnet, dass** das zweite Modul mit einer Durchführungsöffnung und das erste Modul mit einem vorstehenden Stift versehen ist, wobei sich der Stift im Gebrauch durch die Öffnung erstreckt.

8. Messvorrichtung nach Anspruch 7, **dadurch gekennzeichnet, dass** eine Innenfläche der Öffnung und eine Außenfläche des Stifts eine solche Form haben, dass, wenn eine axiale Achse des Stifts und eine axiale Achse der Öffnung zusammenfallen, der Stift nur innerhalb wenigstens eines begrenzten kontinuierlichen Bereichs von Drehwinkeln um seine axiale Achse und in Bezug auf die Öffnung, in die Öffnung eingeführt worden sein oder werden kann.

9. Messvorrichtung nach Anspruch 8, **dadurch gekennzeichnet, dass** der Bereich A ist, wobei A weniger als 15 Grad, vorzugsweise weniger als 10 Grad und bevorzugter weniger als 5 Grad beträgt.

10. Messvorrichtung nach Anspruch 8 oder 9, **dadurch gekennzeichnet, dass** die Außenfläche mit wenigstens einer Nut versehen ist, die sich in einer Längsrichtung des Stifts erstreckt, während die Innenfläche mit wenigstens einem Steg versehen ist, der sich im Gebrauch in der Nut erstreckt, und/oder dass die Innenfläche mit wenigstens einer Nut versehen ist, die sich in einer Längsrichtung der Öffnung erstreckt, während die Außenfläche mit wenigstens einem Steg versehen ist, der sich in der Längsrichtung des Stifts erstreckt und der sich im Gebrauch in der Nut erstreckt.

11. Messvorrichtung nach einem der vorhergehenden Ansprüche, **dadurch gekennzeichnet, dass**, wenn das erste und das zweite Modul nach der einen Möglichkeit oder einer der Möglichkeiten miteinander verbunden sind, das Sensorsystem (142) und die Quelle (122) wenigstens im Wesentlichen gegenüberliegend angeordnet sind, so dass die Strahlung der Quelle auf das Sensorsystem (142) gerichtet ist.

12. Messvorrichtung nach einem der vorhergehenden Ansprüche, **dadurch gekennzeichnet, dass** die Messvorrichtung zum pulsierenden Aktivieren der Lichtquelle ausgelegt ist, wobei das Messsignal den Wert der während der pulsierenden Aktivierung gemessenen Intensität angibt, wobei die Messvorrichtung ferner mit Synchronisationsmitteln zum synchronen Aktivieren der Lichtquelle und des zweiten Moduls (14) versehen ist, wobei die Synchronisationsmittel eine Energieübertragungseinheit (126) umfassen, die Teil des ersten und/oder des zweiten Moduls ist, und einen Detektor (148) umfassen, der Teil des anderen Moduls ist, wobei in einem Betriebszustand die Energieübertragungseinheit (126) pulsierend ein elektromagnetisches Feld erzeugt, und der Detektor dieses Feld empfängt und daraus eine Versorgungsspannung zur Verwendung in dem anderen Modul erzeugt.

13. Messvorrichtung nach Anspruch 12, **dadurch gekennzeichnet, dass** die Messvorrichtung so ausgelegt ist, dass das Synchronisationsmittel (125) im Gebrauch die Lichtquelle (122) und die Energieübertragungseinheit (126) pulsierend aktiviert, wobei der Detektor (148) das Sensormittel (142) als Reaktion auf die von der Energieübertragungseinheit (126) empfangene Energie pulsierend aktiviert oder dass das Synchronisationsmittel (145) das Sensormittel (142) und die Energieübertragungseinheit (126) pulsierend aktiviert, wobei der Detektor (148) die Lichtquelle (122) als Reaktion auf die von der Energieübertragungseinheit (126) empfangene Energie pulsierend aktiviert.

14. Messvorrichtung nach Anspruch 12 oder 13, **dadurch gekennzeichnet, dass**, wenn das erste und das zweite Modul gemäß der einen Möglichkeit oder einer der Möglichkeiten miteinander verbunden sind, die Energieübertragungseinheit und der Detektor wenigstens im Wesentlichen einander gegenüberliegen, so dass die Strahlung von der Energieübertragungseinheit auf den Detektor gerichtet ist.

15. System mit einer Messvorrichtung nach einem der vorhergehenden Ansprüche, **dadurch gekennzeichnet, dass** das System ein Werkzeug mit einem ersten Teil und einem zweiten Teil umfasst, die relativ zueinander beweglich verbunden sind, wobei sich das zweite Teil vom ersten Teil aus gesehen entlang einer vorbestimmten Bahn auf das erste Teil zu und vom ersten Teil weg bewegen kann, wobei das System so ausgelegt ist, dass das erste Modul mit dem ersten Teil auf eine eindeutige Weise mechanisch verbunden werden kann und das zweite Modul mit dem zweiten Teil mechanisch eindeutig verbunden werden kann, wobei, wenn das erste Teil und das zweite Teil aufeinander zu bewegt werden, das erste und das zweite Modul (stabil) miteinander verbunden sind, während sie die vorbestimmte gegenseitige Ausrichtung haben; oder die gegenseitige Ausrichtung haben, die in den vorbestimmten Bereich der gegenseitigen Ausrichtungen fällt.

16. System nach Anspruch 15, **dadurch gekennzeichnet, dass** das Werkzeug als Zange ausgeführt ist, bei der der erste Teil und der zweite Teil aufeinander zu bewegt werden können, indem zwei Griffe der Zange aufeinander zu bewegt werden.

17. System nach Anspruch 15 oder 16, **dadurch gekennzeichnet, dass** das Werkzeug mit einem Sicherungsstift versehen ist und das erste Modul mit einer Sicherungsöffnung versehen ist, in die der Sicherungsstift zur Sicherung des ersten Moduls am Werkzeug eingeführt werden kann.

18. System nach Anspruch 17, **dadurch gekennzeichnet, dass** die Sicherungsöffnung in den Stift des ersten Moduls nach Anspruch 5 hineinragt, um das erste Modul mit dem Werkzeug mechanisch eindeutig zu verbinden.

19. System nach Anspruch 18, **dadurch gekennzeichnet, dass** die Sicherungsöffnung und der Sicherungsstift eine komplementäre Form haben, so dass das erste Modul und das Werkzeug nur auf eine Weise mechanisch miteinander verbunden werden können.

20. System nach einem der Ansprüche 15 bis 19, **dadurch gekennzeichnet, dass** das Werkzeug mit einem teilweise nach außen offenen Hohlraum versehen ist, in dem das zweite Modul aufgenommen werden kann, um das zweite Modul mit dem Werkzeug mechanisch eindeutig zu verbinden.

21. System nach Anspruch 20, **dadurch gekennzeichnet, dass** der Hohlraum und ein Teil einer Außenseite des zweiten Moduls eine komplementäre Form haben, so dass das zweite Modul und das Werkzeug nur auf eine Weise mechanisch miteinander verbunden werden können.

## Revendications

1. Un dispositif de mesure pour mesurer les données physiologiques d'un animal, tel qu'un mammifère, afin de déterminer au moins un paramètre de l'état de l'animal, comprenant un premier et un deuxième module (12, 14) configurés pour, en usage, être portés par l'animal, dans lesquels les premier et deuxième modules sont en outre disposés pour être reliés mécaniquement les uns aux autres, à partir d'un état où ils sont séparés mécaniquement les uns des autres, de sorte que, en cours d'utilisation, à l'état connecté entre le premier et le deuxième module, une partie de la peau de l'animal apparaisse, dans lequel l'un des modules comprend une source (122) d'émission d'un rayonnement électromagnétique, tel que de lumière (L) et dans lequel un autre des modules comprend un système de capteur (142) pour mesurer un paramètre tel que l'intensité d'une fraction (Lm) du rayonnement émis (L) reçu via cette partie de peau et pour générer un signal de mesure (S_{L}) qui est indicatif du paramètre mesuré, dans lequel le dispositif de mesure est configuré de telle sorte que, lors de l'utilisation, le premier et le deuxième module puissent être connectés l'un à l'autre de manière stable seulement lorsqu'ils ont au moins une orientation mutuelle qui est située dans une plage prédéterminée d'orientations mutuelles, **caractérisé en ce que** la plage est limitée à une rotation limitée autour d'un axe qui est au moins sensiblement perpendiculaire à un plan qui, en utilisation, s'étend parallèlement aux côtés des premier et deuxième modules qui se font face.

2. Le dispositif de mesure selon la revendication 1, **caractérisé en ce que** le premier et le deuxième module peuvent éventuellement avoir été connectés ou être connectés entre eux (de manière stable) seulement lorsqu'ils ont une orientation mutuelle qui s'inscrit dans la gamme prédéterminée d'orientations mutuelles

3. Le dispositif de mesure selon la revendication 1 ou 2, **caractérisé en ce que** la rotation limitée implique un angle maximal A où A est inférieur à 15 degrés, de préférence inférieur à 10 degrés et encore de préférence inférieur à 5 degrés.

4. Le dispositif de mesure selon l'une quelconque des revendications précédentes, **caractérisé en ce que** le dispositif de mesure est configuré de telle sorte que si le premier et le deuxième module, en utilisation, ont été fixés l'un à l'autre, le premier et le deuxième module puissent se déplacer l'un par rapport à l'autre selon une possible translation limitée le long d'un axe qui est au moins sensiblement perpendiculaire à un plan qui, en utilisation, s'étend parallèlement aux côtés des premier et deuxième modules qui se font face.

5. Le dispositif de mesure selon la revendication 1 ou 2, **caractérisé en ce que** la possible translation limitée est un maximum de B cm où B est inférieur à 6 cm, de préférence inférieur à 4 cm et de préférence inférieur à 2 cm.

6. Le dispositif de mesure selon l'une des revendications ci-dessus, **caractérisé en ce que** le premier et le deuxième module sont équipés de moyens d'auto-localisation qui assurent qu'en connectant le premier et le deuxième module, le premier et le deuxième module soient tournés les uns par rapport aux autres dans une direction qui correspond à la gamme prédéterminée d'orientations mutuelles.

7. Le dispositif de mesure selon l'une quelconque des revendications précédentes, **caractérisé en ce que** le deuxième module est pourvu d'une ouverture traversante et le premier module est pourvu d'une goupille en saillie, tandis qu'en utilisation, la goupille s'étend dans l'ouverture.

8. Le dispositif de mesure selon la revendication 7, **caractérisé en ce qu'**une surface intérieure de l'ouverture et une surface extérieure de la goupille sont telles que lorsqu'un axe axial de la goupille et un axe axial de l'ouverture coïncident, la goupille peut avoir été insérée ou ne sera insérée dans l'ouverture que dans une plage continue d'angles de rotation limitée autour de son axe axial et par rapport à l'ouverture.

9. Le dispositif de mesure selon la revendication 8, **caractérisé en ce que** la plage est A, où A est inférieur à 15 degrés, de préférence inférieur à 10 degrés, et plus préférablement inférieur à 5 degrés.

10. Le dispositif de mesure selon la revendication 8 ou 9, **caractérisé en ce que** la surface externe est pourvue d'au moins une rainure qui s'étend selon une direction longitudinale de la goupille tandis que la surface interne est pourvue d'au moins une nervure qui, en utilisation, s'étend dans la rainure et/ou que la surface intérieure est pourvue d'au moins une rainure qui s'étend dans le sens de la longueur de l'ouverture tandis que la surface extérieure est pourvue d'au moins une nervure qui s'étend dans le sens de la longueur de la goupille et qui, en utilisation, s'étend dans la rainure.

11. Le dispositif de mesure selon l'une quelconque des revendications précédentes, **caractérisé en ce que** lorsque le premier et le deuxième module ont été connectés l'un à l'autre selon la possibilité unique ou l'une des possibilités, le système de capteur (142) et la source (122) sont situés au moins sensiblement en face l'un de l'autre, de sorte que le rayonnement de la source est dirigé vers le système de capteurs (142).

12. Le dispositif de mesure selon l'une quelconque des revendications précédentes, **caractérisé en ce que** le dispositif de mesure est configuré pour activer de manière impulsionnelle la source lumineuse, dans lequel le signal de mesure est indicatif de la valeur de l'intensité mesurée lors de l'activation de manière impulsionnelle, dans lequel la mesure est en outre pourvue de moyens de synchronisation pour activer de manière synchrone la source lumineuse et le deuxième module (14), dans lequel les moyens de synchronisation comprennent une unité de transmission d'énergie (126) qui fait partie de l'un des premier et deuxième module et comprend un détecteur (148) qui fait partie de l'autre des premier et deuxième module, dans lequel, dans une condition d'utilisation, l'unité de transmission d'énergie (126) génère de manière impulsionnelle un champ électromagnétique, et le détecteur reçoit ce champ et génère à partir de celui-ci une tension d'alimentation à utiliser dans cet autre module.

13. Le dispositif de mesure selon la revendication 12, **caractérisé en ce que** le dispositif de mesure est configuré de telle sorte que, lors de l'utilisation, les moyens de synchronisation (125) activent de manière impulsionnelle la source de lumière (122) et l'unité de transmission d'énergie (126), dans lequel le détecteur (148) active de manière impulsionnelle les moyens de capteur (142) en réponse à l'énergie reçue de l'unité de transmission d'énergie (126) ou que les moyens de synchronisation (145) activent de manière impulsionnelle les moyens de capteur (142) et l'unité de transmission d'énergie (126), dans lequel le détecteur (148) active de manière impulsionnelle la source de lumière (122) en réponse à l'énergie reçue de l'unité de transmission d'énergie (126).

14. Le dispositif de mesure selon la revendication 12 ou 13, **caractérisé en ce que** lorsque le premier et le deuxième module ont été connectés l'un à l'autre selon la possibilité unique ou l'une des possibilités, l'unité de transmission d'énergie et le détecteur sont situés au moins sensiblement en face l'un de l'autre, de sorte que le rayonnement provenant de l'unité de transmission d'énergie est dirigé vers le détecteur.

15. Un système comprenant un dispositif de mesure selon l'une des revendications précédentes, **caractérisé en ce que** le système comprend un outil comprenant une première partie et une deuxième partie connectées de manière mobile les unes par rapport aux autres, dans lequel, vue de la première partie, la deuxième partie peut se déplacer le long d'un chemin prédéterminé vers la première partie et s'éloigner de la première partie, le système étant configuré de telle sorte que le premier module puisse être raccordé mécaniquement à la première partie de manière non équivoque et que le deuxième module puisse être raccordé mécaniquement à la deuxième partie de manière non équivoque, dans lequel, si la première partie et la deuxième partie sont rapprochées l'une à l'autre, le premier et le deuxième module sont (de façon stable) reliés les uns aux autres alors qu'ils ont l'orientation mutuelle prédéterminée; ou ont une orientation mutuelle qui s'inscrit dans la gamme prédéterminée des orientations mutuelles

16. Le système selon la revendication 15, **caractérisé en ce que** l'outil est réalisé sous la forme d'une paire de pinces dont la première partie et la deuxième partie peuvent être rapprochées l'une de l'autre en rapprochant deux poignées de la pince l'une de l'autre.

17. Le système selon la revendication 15 ou 16, **caractérisé en ce que** l'outil est pourvu d'une goupille de fixation et le premier module est pourvu d'une ouverture de fixation dans laquelle la goupille de fixation peut être insérée pour fixer le premier module à l'outil.

18. Le système selon la revendication 17, **caractérisé en ce que** l'ouverture de fixation s'étend dans la goupille du premier module selon la revendication 5 pour relier mécaniquement, de manière non équivoque, le premier module à l'outil.

19. Le système selon la revendication 18, **caractérisé en ce que** l'ouverture de fixation et la goupille de fixation ont une forme complémentaire de sorte que le premier module et l'outil ne peuvent être reliés mécaniquement l'un à l'autre que d'une seule manière.

20. Le système selon l'une quelconque des revendications 15 à 19, **caractérisé en ce que** l'outil est pourvu d'un espace creux partiellement ouvert vers l'extérieur dans lequel peut être reçu le deuxième module pour relier mécaniquement, de manière non équivoque, le deuxième module à l'outil.

21. Le système selon la revendication 20, **caractérisé en ce que** l'espace creux et une partie d'un côté extérieur du deuxième module ont une forme complémentaire de sorte que le deuxième module et l'outil peuvent être mécaniquement reliés l'un à l'autre d'une seule manière.
